(19) **Europäisches Patentamt**
**European Patent Office**
**Office européen des brevets**

(11) **EP 2 438 980 A1**

(12) **EUROPÄISCHE PATENTANMELDUNG**

(43) Veröffentlichungstag:
**11.04.2012 Patentblatt 2012/15**

(51) Int Cl.:
***B01J 8/06*** *(2006.01)* ***C02F 11/04*** *(2006.01)*
***C07C 29/152*** *(2006.01)* ***C07C 31/04*** *(2006.01)*
***C02F 3/30*** *(2006.01)* ***C12P 5/02*** *(2006.01)*

(21) Anmeldenummer: **11177709.0**

(22) Anmeldetag: **16.08.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **01.02.2011 EP 11152947**
**22.02.2011 EP 11155310**
**26.05.2011 EP 11167622**
**06.10.2010 PCT/EP2010/064948**

(71) Anmelder: **Silicon Fire AG**
**6045 Meggen (CH)**

(72) Erfinder: **Meyer-Pittroff, Roland**
**85354 Freising (DE)**

(74) Vertreter: **OK pat AG**
**Chamerstrasse 50**
**6300 Zug (CH)**

(54) **Verfahren und Vorrichtung zur Bereitstellung und zum Einsetzen von wasserstoff-basiertem Methanol zu Denitrifizierungszwecken**

(57) Verfahren und Vorrichtung zum Bereitstellen und Einsetzen einer methanolhaltigen Flüssigkeit (108) mit den folgenden Schritten:
- Bereitstellen (52; 104) eines Kohlenstoffdioxidgases (101) als Kohlenstofflieferant,
- Bereitstellen eines Wasserstoffgases (103),
- Bereitstellen eines gasförmigen Ausgangsstoffes (AS), der das Kohlenstoffdioxidgas (101) und Wasserstoffgas (103) umfasst,
- Einbringen des Ausgangsstoffes (AS) in einen Reaktor (10),
- Durchlaufen des Ausgangsstoffes (AS) durch eine Reaktionsstrecke des Reaktors (10), die mindestens teilweise mit einem Katalysator bestückt ist, um auf katalysator-synthetischem Weg die methanolhaltige Flüssigkeit (108) zu synthetisieren,
- Bereitstellen der methanolhaltigen Flüssigkeit (108) an einem ausgangsseitigen Ende (23) des Reaktors (10), wobei es sich bei der methanolhaltigen Flüssigkeit (108) um ein Gemisch (108) aus Methanol und Wasser handelt,
- Einsetzen der methanolhaltigen Flüssigkeit (108) in einem Denitrifizierungsprozess (600), um einen Stickstoffanteil aus Wasser, vorzugsweise aus Abwasser, zu entfernen.

Fig. 4

**Beschreibung**

Die vorliegende Anmeldung betrifft Verfahren und Vorrichtung zur Bereitstellung und zum Einsetzen von wasserstoff-basiertem Methanol zu Denitrifizierungszwecken.

Die vorliegende Anmeldung beansprucht die Priorität der internationalen Patentanmeldung PCT/EP2010/064948, die am 6. Oktober 2010 unter dem Titel "VERFAHREN UND ANLAGE ZUR SYNTHESE VON KOHLENWASSERSTOFF" eingereicht wurde.

Die vorliegende Anmeldung beansprucht auch die Priorität

- der Europäischen Patentanmeldung EP11152947.5, die am 1. Februar 2011 unter dem Titel "VERFAHREN ZUR BEREITSTELLUNG UND ZUM EINSETZEN EINES ALKOHOLS UND VERWENDUNG DES ALKOHOLS ZUR LEISTUNSSTEIGERUNG EINER VERBRENNUNGSKRAFTMASCHINE",
- der Europäischen Patentanmeldung EP11155310.3, die am 22. Februar 2011 unter dem Titel "Verfahren zur Bereitstellung und zum Einsetzen eines Alkohols und Verwendung des Alkohols zur Wirkungsgrad- und Leistungssteigerung einer Verbrennungskraftmaschine", und
- der Europäischen Patentanmeldung EP11167622.7, die am 26. Mai 2011 unter dem Titel "VERFAHREN UND VORRICHTUNG ZUR BEREITSTELLUNG UND ZUM EINSETZEN VON WASSERSTOFF-BASIERTEM METHANOL ZU DENITRIFIZIERUNGSZWECKEN"

eingereicht wurden.

Kohlenstoffdioxid $CO_2$ (meist Kohlendioxid genannt) ist eine chemische Verbindung aus Kohlenstoff und Sauerstoff. Kohlendioxid ist ein farb- und geruchloses Gas. Es ist mit einer geringen Konzentration ein natürlicher Bestandteil der Luft und entsteht in Lebewesen bei der Zellatmung, aber auch bei der Verbrennung von kohlenstoffhaltigen Substanzen bei ausreichender Anwesenheit von Sauerstoff. Seit Beginn der Industrialisierung steigt der $CO_2$-Anteil in der Atmosphäre deutlich an. Hauptursache hierfür sind die vom Menschen verursachten - sogenannten anthropogenen - $CO_2$-Emissionen. Das Kohlendioxid in der Atmosphäre absorbiert einen Teil der Wärmestrahlung. Diese Eigenschaft macht Kohlendioxid zu einem so genannten Treibhausgas (THG) und zu einem der Mitverursacher des globalen Treibhauseffekts.

Aus diesen und auch aus anderen Gründen wird zur Zeit in verschiedenste Richtungen geforscht und entwickelt, um einen Weg zu finden, um die anthropogenen $CO_2$-Emissionen zu reduzieren. Besonders im Zusammenhang mit der Energieerzeugung, die häufig durch das Verbrennen fossiler Energieträger, wie Kohle, Öl oder Gas, erfolgt, aber auch mit anderen Verbrennungsprozessen, zum Beispiel der Müllverbrennung, besteht ein großer Bedarf zur Reduktion der $CO_2$-Emission. Es werden z.Z. pro Jahr ca. 30 Milliarden Tonnen $CO_2$ durch solche Prozesse in die Atmosphäre abgegeben.

Es wird als Problem angesehen, dass bei der Verbrennung von fossilen Energieträgern $CO_2$ entsteht. Ausserdem werden die fossilen Ressourcen, die endlich sind, unwiderruflich verbraucht. Insbesondere die Mobilität ist mit größeren Emissionen verbunden. Daher wird in verschiedenste Richtungen geforscht, um den Verbrauch von Fahrzeugen zu senken oder um Fahrzeuge zu entwickeln, die komplett mit regenerativen Energien angetrieben werden.

Neben den Problemen der Luftverschmutzung und -belastung gibt es auch Probleme im Zusammenhang mit der Gewässerverschmutzung. Unter anderem tritt Stickstoff in den Gewässern sowohl molekular als Stickstoff (N2) als auch in anorganischen und organischen Verbindungen auf. Harnstoff ist die größte Stickstoffquelle im kommunalen Abwasser. Der Grenzwert für Trinkwasser liegt ja nach der lokal gültigen Trinkwasserverordnung z.B. bei 50 mg/l Nitrat, was stöchiometrisch ca. 11 mg/l Nitrat-Stickstoff entspricht.

Es ist bekannt, dass man den Stickstoffanteil im Abwasser durch Denitrifizierungsverfahren (auch Denitrifikation oder Stickstoffelimination genannt) reduzieren kann. Bei der Denitrifikation werden Nitrate zu sauerstoffarmen Stickstoffverbindungen und dann zu elementarem, gasförmigem Stickstoff reduziert. Im Rahmen der Denitrifikation braucht es ein biologisch abbaubares organisches Substrat. Wenn nicht genügend Substrat vorhanden ist, wird z.B. Methanol zugeführt.

Methanol ist ein besonders vorteilhaftes Substrat, da es vollständig in Wasser löslich und leicht biologisch abbaubar ist. Methanol wird jedoch bisher in den meisten Fällen aus fossilen Rohstoffen, zum Beispiel aus Erdgas, hergestellt. Es sind zahlreiche Verfahren und Reaktoren zur Herstellung von Methanol bekannt. Im Folgenden sind entsprechende beispielhafte Patentanmeldungen und Patente genannt:

- EP 0 790 226 B1;
- WO 2010/037441 A1;
- EP 4 483 919 A2.

Es besteht für die eingangs erwähnten Anwendungen der Bedarf für die Bereitstellung von Methanol, das

CO$_2$-neutral und günstig in der Herstellung ist. Ausserdem soll die Methanolherstellung nicht mit der Nahrungsmittelproduktion in Konkurrenz stehen und keinen Flächenbedarf haben wie bei der Herstellung aus Biomasse.

Es stellt sich nun die Aufgabe, ein entsprechendes Verfahren und eine entsprechende Vorrichtung zur Bereitstellung von Methanol speziell zu Denitrifikationszwecken in Abwasserbehandlungsanlagen zu entwickeln, das ökologisch und ökonomisch sinnvoll ist.

Gemäss Erfindung wird daher eine neue Verfahrenskette vorgeschlagen, bei der es um das Bereitstellen und Verbrauchen von Methanol in einer Abwasserbehandlungsanlage geht.

Das Verfahren umfasst die folgenden Schritten:

- Bereitstellen eines Gases mit Kohlendioxidanteil (CO$_2$) als Kohlenstofflieferant,
- Bereitstellen eines Wasserstoffanteils (H$_2$),
- Bereitstellen eines Ausgangsstoffes, der den Kohlendioxidanteil und Wasserstoffanteil umfasst,
- Einbringen des Ausgangsstoffes in einen Reaktor,
- Durchlaufen des Ausgangsstoffs durch eine Reaktionstrecke des Reaktors, die mindestens teilweise mit einem Katalysator bestückt ist, um in einem synthesekatalytischen Verfahren Methanol zu synthetisieren,
- Bereitstellen von Methanol an einem ausgangsseitigen Ende des Reaktors, wobei vorzugsweise ein Gemisch aus einem Methanolanteil und einem Wasseranteil vorhanden ist,
- Einsetzen dieses Methanols in einer Abwasserbehandlungsanlage zu Denitrifikationszwecken.

Ziel der Erfindung ist es auch, ein System bereit zu stellen, das möglichst autark, d.h. weitestgehend netzunabhängig funktioniert. Insbesondere geht es daher hier um ein Verfahren, bei dem mindestens ein Teil des Strombedarfs, der besteht, um den Wasserstoffanteil (H$_2$) elektrolytisch bereit zu stellen, lokal in der Abwasserbehandlungsanlage oder in deren unmittelbaren Umfeld erzeugt wird.

Vorzugsweise wird der Strom erzeugt, indem Abfallstoffe, die in einer Kläranlage anfallen, verbrannt werden, um einen Stromgenerator betreiben zu können, oder indem Abfallstoffe zum Beispiel zu methanhaltigem Gas umgesetzt und dieses Gas zur Stromerzeugung eingesetzt wird.

Alternativ kann der Wasserstoffanteil (H$_2$) auch z.B. direkt aus methanhaltigem Gas erzeugt werden. In diesem Fall ist keine Elektrolyse zur Bereitstellung des Wasserstoffanteils (H$_2$) erforderlich.

Es ist auch eine Kombination aus Elektrolyseanlage und Wasserstoffbereitstellung aus lokal vorhandenem Gas möglich.

Die Erfindung setzt bewusst auf Kohlendioxid und Wasserstoff als Ausgangsstoffe, da das Kohlendioxid auf diesem Wege "rezykliert" werden und über den Einsatz von Methanol als Kohlenstofflieferant für die Denitrifizierung dienen kann.

Wasserstoff kann zusätzlich oder alternativ auch aus regenerativer Energie erzeugt und das Kohlendioxid aus Abgasen gewonnen oder aus Biomasse erzeugt werden, damit das Methanol, das aus diesen Ausgangsstoffen auf katalytischem Weg synthetisiert wird, als CO$_2$-neutral angesehen werden kann. Ausserdem hat dieser Weg den Vorteil, dass er z.B. bei Synthese von Methanol ein Methanol-Wasser-Gemisch liefert, das direkt zum Denitrifizieren geeignet ist. Die Zusammensetzung des Methanol-Wasser-Gemischs ist ideal, um als für das Denitrifizieren eingesetzt zu werden, und sie zeichnet sich dadurch aus, dass sie auch aus umwelttechnischer Sicht Vorteile hat.

Anders als bei bisherigen Methanol-Produktionsverfahren kann hier das Produkt des Methanol-Syntheseprozesses, bestehend aus Methanol und Wasser, unmittelbar als Flüssigkeit für die Denitrifikation eingesetzt werden. Es braucht keinen weiteren Energieaufwand zum Destillieren des Produkts des Syntheseprozesses. Der Energieaufwand zum Destillieren, der typischerweise betrieben wird, um reines hochkonzentriertes Methanol zu erhalten, führt zu einer Verteuerung des Methanols.

Gemäss Erfindung wird H$_2$- und CO$_2$-haltiges Synthesegas effizient und wirtschaftlich sinnvoll zu Methanol für Denitrifikationszwecke umgesetzt.

Gemäß Erfindung wird Kohlendioxid auf dem Umweg über Methanol als Kohlenstofflieferant für Denitrifikationszwecke eingesetzt. Das Kohlendioxid wird mit dem Wasserstoffanteil in Anwesenheit eines Katalysators zur Reaktion gebracht, um es zu einem Methanol-Wasser-Gemisch umzusetzen.

Vorzugsweise wird Kohlendioxid aus einem Verbrennungsprozess oder einem Oxidationsprozess von Kohlenstoff oder Kohlenwasserstoffen mittels CO$_2$-Abscheidung entnommen. CO$_2$ kann zum Beispiel aus einem Klärprozess stammen. Wenn das CO$_2$ aus einem Klärprozess stammt, ist die erfindungsgemässe Anlage autark, da je nach Auslegung der Anlage und je nach Umgebungsbedingungen weder Energie noch andere Stoffe von extern angeliefert oder zugeführt werden müssen.

Das erfindungsgemäße Verfahren zur Bereitstellung der Denitrifizierungsflüssigkeit wird so gesteuert und die einzelnen Prozesse werden so miteinander "verknüpft", dass

- der Gesamtertrag und die Qualität des Methanols ideal für den Einsatzzweck ist,

- und/oder die CO$_2$-(Gesamt-)Emission möglichst minimal wird,
- und/oder eine möglichst konstante und langzeitige Anlagenauslastung erzielt wird,
- und/oder die produktspezifischen Investitions- und Betriebskosten möglichst minimal werden.

Vorzugsweise wird lokal vorhandene Energie und/oder regenerative elektrische Energie zum Bereitstellen des Methanols eingesetzt.

Mit einer entsprechenden Anlage wird vorzugsweise ein Methanol-Wasser-Gemisch als speicher- und transportierbare Flüssigkeit hergestellt. D.h., es werden die lokal vorhandene Energie und/oder die erneuerbare Energie auf chemischem Wege in eine unkritische und relativ einfach speicher- und transportierbare Flüssigkeit überführt.

Die Produktion der Flüssigkeit als relativ einfach speicher- und transportierbares Gemisch kann jederzeit heruntergefahren oder gar unterbrochen werden. Die verfahrenstechnischen Anlagenteile zur Herstellung des Gemischs können relativ einfach und schnell heruntergefahren oder abgeschaltet werden. Hier liegt die Entscheidungshoheit im Verantwortungsbereich des Betreibers der Anlage.

Bevorzugte Ausführungsformen der Erfindung basieren auf der Wasserstofferzeugung mit Hilfe elektrischer Energie, die weitmöglich lokal (d.h. vor Ort im Bereich der Abwasserbehandlungsanlage) regenerativ erzeugt wird und z.B. aus Biomasse-, Biogas-, Faulgas-, Wind-, Wasser-, Geothermie- und/oder Solarkraftwerken stammt. Wasserstoff, der z.B. vor Ort per Elektrolyse oder aus Abfallstoffen erzeugt wird, braucht also nicht gelagert oder hoch verdichtet oder tief gekühlt verflüssigt und über größere Strecken transportiert zu werden, sondern dient als Zwischenprodukt, das vorzugsweise am Standort seiner Erzeugung unmittelbar oder zeitnah der vorgenannten Reaktion zur Erzeugung von Methanol zugeführt wird.

Das entsprechende Methanol-Wasser-Gemisch kann aber gemäss Erfindung auch unter Einsatz eines intelligenten Energiemixes (wie z.B. in der internationalen Patentanmeldung WO2010069622A1 beschrieben) aus fossiler und regenerativer Energie erzeugt werden.

Unter Beachtung entsprechender energietechnischer, anlagentechnischer und wirtschaftlicher Vorgaben, zusammen mit der Forderung nach schonender Nutzung aller stofflichen, energetischen und ökonomischen Ressourcen, werden gemäß Erfindung ein neues, energietechnisch relevantes Verfahren und eine entsprechende Verwendung bereitgestellt.

Weitere vorteilhafte Ausführungsformen sind der Beschreibung, den Figuren und den abhängigen Ansprüchen zu entnehmen.

In den Zeichnungen sind verschiedene Aspekte der Erfindung schematisch dargestellt.

Fig. 1: zeigt ein Schema, das die grundlegenden Schritte des Verfahrens, gemäß einer der eingangs erwähnten internationalen Patentanmeldungen, respektive einer entsprechenden Silicon-Fire Anlage wiedergibt;

Fig. 2: zeigt ein Schema, das die grundlegenden Schritte des Verfahrens gemäß Erfindung, respektive einer entsprechenden Silicon-Fire Anlage wiedergibt;

Fig. 3: zeigt eine seitliche Außenansicht eines Reaktors, der in einem erfindungsgemässen Verfahren eingesetzt werden kann;

Fig. 4: zeigt eine stark schematisierte Ansicht eines Gesamtabwasserbehandlungsverfahrens gemäss Erfindung;

Fig. 5: zeigt eine stark schematisierte Ansicht eines Gesamtverfahrens gemäss Erfindung zur Nutzung der lokal vorhandenen Ressourcen, um das Verfahren autark zu machen.

Es geht hier um Methanol-Wasser-Gemische 108, die auch als methanolhaltige Flüssigkeit bezeichnet werden.

Der Begriff Gemisch 108 wird hier verwendet, da das Produkt, das am Ausgang 23 eines Reaktors 10 bereit gestellt wird, nicht zu hundert Prozent aus Methanol besteht. Es handelt sich vielmehr um ein sogenanntes physikalisches Gemisch aus Methanol und Wasser.

Der Begriff Abwasserbehandlungsanlage wird hier für jegliche Form von Anlage verwendet, die zur (Ab-) Wasseraufbereitung oder -reinigung einsetzbar ist. Insbesondere geht es um den Einsatz in einer Kläranlage.

Es geht hier speziell um eine Kombination der Methanol-Denitrifizierung und Verwendung von Silicon-Fire-Methanol 108, wobei das benötigte CO$_2$ aus dem Faulgas und/oder aus dem Faulgas-Verbrennungsgas gewonnen und die benötigte elektrische Energie aus einem Faulgas betriebenen Blockheizkraftwerk 602 (Kombination von Verbrennungsmotor mit Generator und Abwärmenutzung z.B. zur Beheizung der Klärbecken) erzeugt wird.

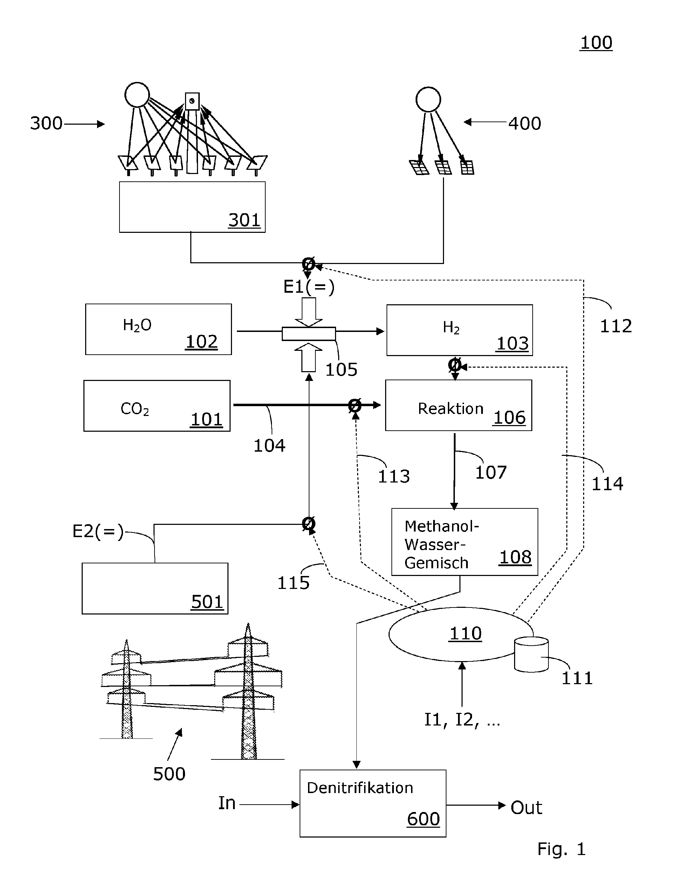

Fig. 1 zeigt in einer schematischen Blockdarstellung die wichtigsten Bausteine/Komponenten, respektive Verfahrensschritte, einer Silicon-Fire Anlage 100 gemäß einer der eingangs erwähnten internationalen Patenanmeldungen. Diese Anlage 100 ist so ausgelegt, dass ein Verfahren zum Bereitstellen der Methanolflüssigkeit 108 ausgeführt werden kann. Das entsprechende Verfahren basiert auf den folgenden grundlegenden Schritten.

Es wird Kohlenstoffdioxid 101 als Kohlenstofflieferant bereitgestellt. Die für das Erzeugen von Wasserstoff 103 erforderliche elektrische Gleichstromenergie E1 wird hier weitmöglich mittels erneuerbarer Energietechnik erzeugt und der Silicon-Fire Anlage 100 zur Verfügung gestellt. Besonders als erneuerbare Energietechnik geeignet sind Solarthermieanlagen 300 und Photovoltaikanlagen 400, die auf Solarmodulen basieren. Als regenerative Energiequellen können z.B. auch Wasserkraft, Windkraft oder Geothermie eingesetzt werden. Die regenerative Energiequellen können z.B. auch biogenen Ursprungs (u.a. Klärschlamm oder Klärgas) sein.

Es wird gemäß Fig. 1 eine Wasserelektrolyse 105 unter Einsatz der elektrischen Gleichstromenergie E1 durchgeführt, um Wasserstoff 103 als Zwischenprodukt zu erzeugen.

Bei der Silicon-Fire Anlage 100 wird vorzugsweise eine wirtschaftlich und ökologisch optimale Kombination regenerativer Stromversorgung (z.B. durch die Anlagen 300 und/oder 400) und konventioneller Stromversorgung, hier durch einen Teil eines Verbundnetzes 500 dargestellt, realisiert. Ein Teil oder die gesamte elektrische Energie kann auch lokal erzeugt werden (z.B. durch Verwendung eines lokal anfallenden Gases oder lokal anfallender Stoffe, die umgesetzt werden können). Diese Silicon-Fire Anlage 100 sieht daher vor, die elektrische Energie E1 weitgehend direkt entsprechend ihrem Anfall für chemische Reaktionen (hier die Elektrolysereaktion 105) zu nutzen und damit chemisch zu binden und zu speichern. Ein weiterer Anteil der benötigten Energie wird hier beispielsweise aus dem Verbundnetz 500 und/oder aus lokalen Anlagen (z.B. einem Generator) bezogen. Dieser Anteil wird in Gleichstrom(energie) E2 umgewandelt. Zu diesem Zweck kommt ein entsprechender Umwandler 501 zum Einsatz, wie in Fig. 1 in schematischer Form angedeutet. Die entsprechenden Anlagenteile oder -komponenten werden hier auch als (lokale) Energieversorgungsanlage 501 bezeichnet.

Mittels einer intelligenten Anlagensteuerung 110 wird die Energieversorgung der Anlage 100 nach Fig. 1 gesteuert und geregelt. Es wird im Prinzip der jeweils momentan verfügbare überschüssige Energieanteil E2 aus dem Verbundnetz 500 bezogen, während der andere Energieanteil (hier E1) soweit möglich aus einem (Anlage-bezogenen) Solarkraftwerk 300 und/oder 400 (und/oder aus einem Windkraftwerk und/oder aus einem Biomassekraftwerk und/oder aus einem Wasserkraftwerk und/oder aus einem Geothermiekraftwerk) bezogen wird. Dieses Prinzip ermöglicht es dem Betreiber einer Silicon-Fire Anlage 100, zusätzliche technische und wirtschaftliche Parameter bei der Steuerung der Anlage 100 einzubeziehen. Bei diesen Parametern handelt es sich um sogenannte Input-Größen I1, I2, usw., die von der Steuerung 110 in Entscheidungen einbezogen werden. Ein Teil der Parameter kann innerhalb der Steuerung 110 in einem Parameterspeicher 111 vorgegeben werden. Ein anderer Teil der Parameter kann von außen kommen. Durch die Steuerung 110 kann das erfindungsgemässe Verfahren in der Anlage 100 so geführt werden, dass die Methanolflüssigkeit 108, die ausgangsseitig bereit gestellt wird, die gewünschten Anforderungen in Bezug auf das Mischungsverhältnis und/oder die $CO_2$ Neutralität erfüllt.

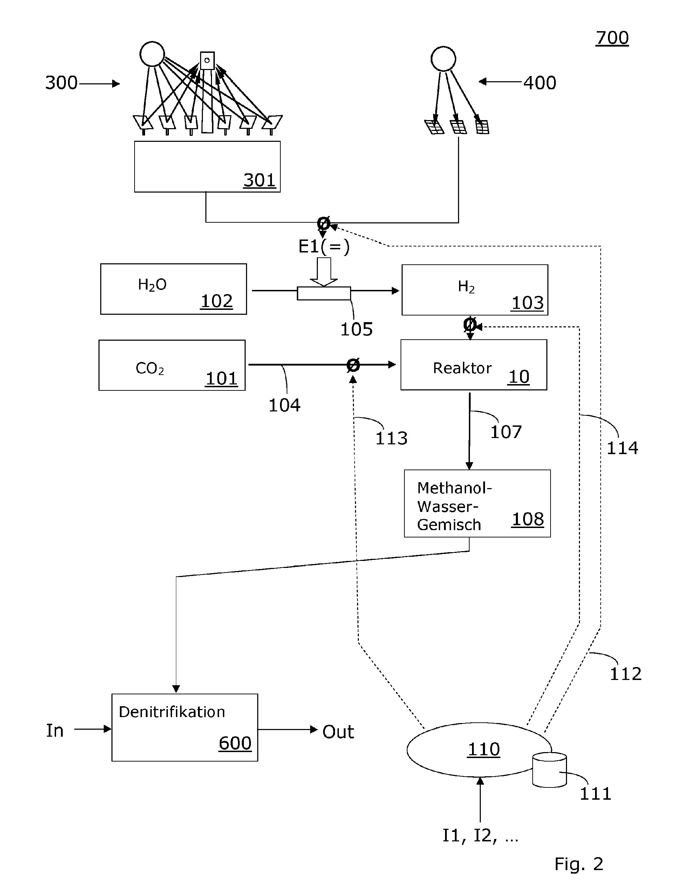

In Fig. 2 ist eine weitere Anlage 700 schematisch dargestellt, die eingesetzt werden kann, um das erfindungsgemässe Verfahren auszuführen. Ein Teil dieser Anlage 700 entspricht der Anlage 100 nach Fig. 1. Es wird daher auf die vorausgehende Beschreibung der entsprechenden Elemente verwiesen.

Es wird auch bei dieser Anlage 700, wie beschrieben, durch eine Wasserelektrolyse 105 hochreiner Wasserstoff 103 erzeugt, der hier zu einem Methanol-Wasser-Gemisch 108 umgesetzt wird. Die Energie hierzu stammt bei dieser Ausführungsform ganz oder weitestgehend (vorzugsweise zu mehr als 80 %) aus regenerativen Energiequellen 300 und/oder 400, oder aus anderen regenerativen Energiequellen und/oder aus oder aus lokalen Energiequellen.

Es können eine Reihe von Steuer- oder Signalleitungen vorgesehen sein, wie anhand der beispielhaft gezeigten Leitungen 112, 113, 114 und 115 dargestellt. Diese Leitungen 112, 113, 114 und 115 steuern Energie- oder Massenströme der Anlage 100 oder 700.

In den Figuren 1 und 2 ist gezeigt, dass das Methanol-Wasser-Gemisch 108 zur Denitrifikation 600 eingesetzt wird. Es wird Wasser (z.B. Abwasser) auf der Eingangsseite (mit IN bezeichnet) zugeführt. Nach dem Ausführen der Denitrifikation 600 wird Wasser, das jetzt weniger Stickstoff enthält, auf der Ausgangsseite (mit OUT bezeichnet) abgegeben. Details dieses Denitrifikationsverfahrens 600 sind hinlänglich bekannt und werden hier nicht im Detail beschrieben.

In der Anlagensteuerung 110 sind sogenannte software-basierte Entscheidungsprozesse implementiert. Ein Prozessor der Steuerung 110 führt eine Steuerungssoftware aus und fällt unter Berücksichtigung von Parametern programmierte Entscheidungen. Diese Entscheidungen werden in Schalt- oder Steuerungsbefehle umgesetzt, die zum Beispiel über Steuer- oder Signalleitungen 112, 113, 114, 115 die Steuerung/Regelung von Energie- und Massenströmen bewirken. Durch die Steuerung 110 kann das Verfahren in der Anlage 100 so geführt werden, dass die Flüssigkeit 108, die ausgangsseitig bereit gestellt wird, die gewünschten Anforderungen in Bezug auf das Mischungsverhältnis und/oder die $CO_2$-Neutralität erfüllt.

Gemäß Erfindung wird Kohlendioxid 101 als gasförmiger Kohlenstofflieferant eingesetzt, wie in Fig. 1 und Fig. 2 schematisch angedeutet. Vorzugsweise wird das Kohlendioxid 101 aus einem Verbrennungsprozess oder einem Oxidationsprozess über $CO_2$-Abscheidung (z.B. eine Silicon-Fire Rauchgasreinigungsanlage) entnommen. Das Kohlendioxid 101 kann aber auch aus einer Kläranlage bereitgestellt werden. Das Kohlendioxid 101 kann auch aus anderen Quellen kommen.

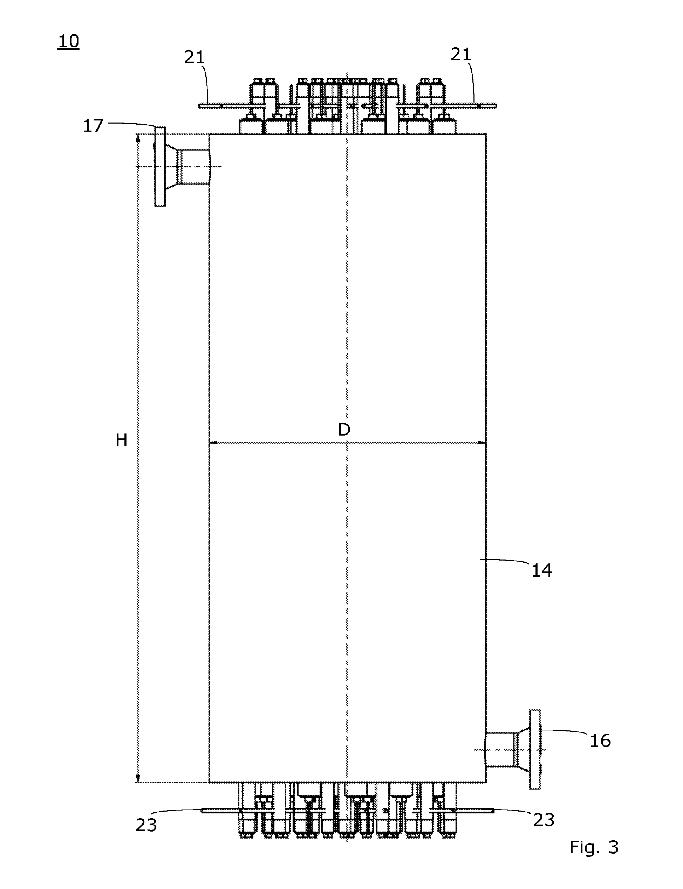

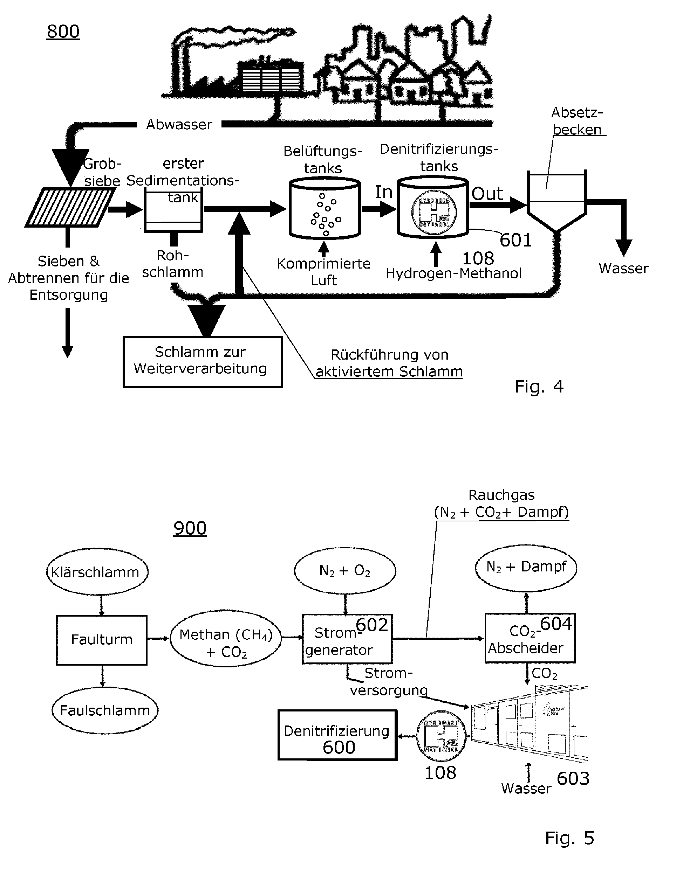

Weiterhin wird bei der in Fig. 2 gezeigten Anlage 700 elektrische Gleichstromenergie E1 bereitgestellt (das gleiche gilt auch für die Anlage in Fig. 4). Die Gleichstromenergie E1 wird vorzugsweise weitgehend regenerativ (z.B. durch eine der Anlagen 300 und/oder 400 in Fig. 2) und/oder auf anderem Wege lokal erzeugt. Die Gleichstromenergie E1 wird bei der gezeigten Anlage 700 zum Durchführen einer Wasserelektrolyse eingesetzt, um Wasserstoff 103 als Zwischenprodukt zu erzeugen. Die Elektrolyseanlage, respektive das Durchführen einer solchen Elektrolyse, ist in Fig. 1, Fig. 2 und Fig. 4 durch das Bezugszeichen 105 gekennzeichnet. Das Kohlendioxid 101 wird mit dem Wasserstoff 103 zusammen geführt. Das entsprechende Gas wird hier als Ausgangsstoff AS bezeichnet. Der Ausgangsstoff AS wird zur Reaktion (Methanolsynthese in einem Reaktor 10, wie z.B. in Fig. 3 gezeigt) gebracht, um die gasförmigen (Zwischen-) Produkte 101, 103 zu dem Methanol-Wasser-Gemisch 108 umzusetzen. Die Reaktion wird in dem Reaktor 10 durchgeführt. Die Entnahme, respektive das Bereitstellen des Methanol-Wasser-Gemischs 108, ist in Fig. 1 und Fig. 2 durch das Bezugszeichen 107 gekennzeichnet.

Um Wasserstoff 103 als Zwischenprodukt erzeugen zu können, eignet sich eine Wasserelektrolyse unter Einsatz von Gleichstrom E1. Der benötigte Wasserstoff 103 wird in einer Elektrolyseanlage 105 durch die Elektrolyse von Wasser $H_2O$ nach folgender Gleichung hergestellt:

$$H_2O - 286,02 \text{ kJ} = H_2 + 0,5 \text{ } O_2. \qquad \text{(Reaktion 1)}$$

Die benötigte (elektrische) Energie E1 für diese Reaktion von 286,02 kJ/mol entspricht 143010 kJ pro kg $H_2$.

Die Synthese des Methanol-Wasser-Gemischs 108 ($CH_3OH$) kann in dem Reaktor 10 der Silicon-Fire Anlage 700 nach der exothermen Reaktion zwischen Kohlendioxid 101 ($CO_2$) und Wasserstoff 103 ($H_2$) wie folgt erfolgen:

$$CO_2 + 3 \text{ } H_2 = CH_3OH + H_2O - 49,6 \text{ kJ} \text{ (Methanol-Wassergemisch, dampfförmig)}$$

$$\text{(Reaktion 2)}$$

Die entstehende Reaktionswärme von 49,6 kJ/mol = 1550 kJ pro kg Methanol = 0,43 kWh pro kg Methanol 108 wird aus dem entsprechenden Reaktor 10 abgeführt. Zu diesem Zweck umfasst der Reaktor 10 einen Fluidraum 14 (siehe z.B. Fig. 3), d.h. der Reaktor 10 ist von einem Reaktormantel umgeben und durch ein Fluid (vorzugsweise Wasser) gekühlt.

Typische Synthesebedingungen im Synthesereaktor 10 sind ca. 50 bis 80 bar und ca. 270 °C. Die Reaktionswärme kann z.B. an andere Anlagenelemente "übergeben" und dadurch genutzt werden.

Die Methanol-Wasser-Synthese wird gemäss Erfindung unter Einsatz eines Katalysators 60 durchgeführt, um Reaktionstemperatur, Reaktionsdruck sowie Reaktionsdauer im Vergleich zu anderen Verfahren niedrig zu halten und um sicher zu stellen, dass als Reaktionsprodukt ein flüssiges Methanol-Wasser-Gemisch 108 entsteht, das als Denitrifizierungsflüssigkeit geeignet ist.

In Fig. 2 ist anhand des gestrichelten Pfeils 112 angedeutet, der von der Steuerung 110 ausgeht, dass die Steuerung 110 den Energiestrom E1 regelt. Der Pfeil 112 stellt eine Steuer- oder Signalleitung dar. Es sind auch andere mögliche Steuer- oder Signalleitungen 113, 114 dargestellt. Die Steuer- oder Signalleitung 113 regelt zum Beispiel die $CO_2$-Menge, die für die Reaktion 106 zur Verfügung steht. Wenn zum Beispiel weniger Wasserstoff 103 produziert wird, dann muss auch proportional weniger $CO_2$ zugeführt werden. Die optionale Steuer- oder Signalleitung 114 kann zum Beispiel die $H_2$-Menge regeln. Eine solche Regelung ist dann sinnvoll, wenn es einen Wasserstoff-Pufferspeicher gibt, dem man Wasserstoff 103 entnehmen kann, auch wenn im Moment kein Wasserstoff oder weniger Wasserstoff durch Elektrolyse 105 produziert wird.

Details einer besonders bevorzugten Ausführungsform eines Reaktors 10 zur Synthese des Methanol-Wasser-Gemischs 108 ist in den Fig. 3 gezeigt. Die Ausführungen, die in der internationalen Patentanmeldung PCT/EP2010/064948, Anmeldedatum 6. Oktober 2010, zur Synthese von Methanol 108 gemacht werden, lassen sich auch auf die Synthese anderer flüssiger Kohlenwasserstoffe übertragen.

Das Methanol-Wasser-Gemisch 108 wird, wie bereits beschrieben, unter Einsatz eines Ausgangsstoffes AS, der $CO_2$-Gas 101 und Wasserstoffgas 103 enthält, synthetisiert. Der entsprechende Reaktor 10 umfasst ein Reaktorelement oder mehrere parallel zueinander angeordnete Reaktorelemente. Am Reaktor 10 gibt es mindestens einen Gaseintritt 21 für den Ausgangsstoff AS und einen Produktauslass 23, wie in Fig. 3 beispielhaft gezeigt.

Der Ausgangsstoff AS wird beim Durchlaufen respektive beim Hindurchpressen durch die Reaktorröhre(n) des Reaktors 10 sukzessive zu einem methanolhaltigen Gemisch 108 (alkoholhaltige Kühlflüssigkeit genannt) umgesetzt. Auf der Eingangsseite des Reaktors 10 liegt die Methanolkonzentration des Reaktionsfluids bei Null und die Konzentration des gasförmigen Ausgangsstoffes AS bei ca. 100 %. In Richtung Ausgangsseite des Reaktors 10 verschieben sich die entsprechenden Konzentrationen gegenläufig bis am Ausgang (am Produktauslass 23) ein methanolhaltiges Gemisch 108 mit einer vorgegebenen Methanolkonzentration (vorzugsweise ein Methanol-Wasser-Gemisch im Verhältnis 1:2) gebildet ist.

Der Reaktor 10 liefert als Rohmethanol ca. 64 Mass.-% (69,2 Vol.-%) Methanol und 36 Mass.-% (30,8 Vol.-%) Wasser.

Der Reaktor 10 oder die Reaktorelemente des Reaktors 10 umfassen bei allen Ausführungsformen einen Katalysator für die Synthese des Methanol-Wasser-Gemischs 108.

Vorzugsweise kommt bei allen Ausführungsformen eine Steuerung des Reaktors 10 zum Einsatz, die anfangs beim "Hochfahren" des Reaktors 10 den Fluidraum 14 mit warmem Fluid beaufschlagt, um die Synthesereaktion in Gang zu setzen. Anschliessend wird vorzugsweise ein gekühltes Fluid zugeführt, um Reaktionswärme, die bei der exothermen Synthese entsteht, abzuführen und um so eine isotherme Umgebung zu schaffen.

Vorzugsweise ist der Fluidraum 14 bei allen Ausführungsformen so ausgelegt, dass mindestens die Reaktionsabschnitte des Reaktors 10, die mit dem Katalysator gefüllt sind, in der isothermen Umgebung liegen.

In Fig. 3 ist der Reaktor 10 von aussen gezeigt.

Vorzugsweise wird bei allen Ausführungsformen der Erfindung der Ausgangsstoff AS vorgewärmt und/oder mit erhöhtem Druck durch Zufuhrleitungen in den Reaktor 10 eingebracht. Der Druck und die Temperatur hängen von der Art des Katalysators ab. Vorzugsweise liegt die Temperatur im Bereich zwischen 100 und 350 °C. Der Druck liegt typischerweise zwischen 10 und 150 bar. Daher kann auch gesagt werden, dass der Ausgangsstoff AS vorzugsweise bei allen Ausführungsformen unter Vorgabe eines eingangsseitigen Drucks zwischen 10 und 150 bar durch den Reaktor 10 gepresst wird.

Der Reaktor 10 eignet sich speziell für die Synthese eines regenerativen Methanol-Wasser-Gemischs 108 aus Kohlendioxid $CO_2$ und Wasserstoff $H_2$, der über die (endotherme) Elektrolyse von Wasser mit regenerativer elektrischer Energie E1 gemäß Reaktion 1, wie bereits weiter oben erwähnt, erzeugt wird.

$$H_2O - 286,02 \text{ kJ/mol} = H_2 + 0,5O_2 . \qquad \text{(Reaktion 1)}$$

Die exotherme Methanol-Wasser-Synthese (Reaktion 2, wie weiter oben bereits erwähnt) wird durch die Summenformel dargestellt:

$$CO_2 + 3 H_2 = CH_3OH + H_2O - 49,6 \text{ kJ (Methanol gasförmig)}. \qquad \text{(Reaktion 2)}$$

Es muss betont werden, dass in allen Ausführungsformen natürlich auch andere Syntheseverfahren und andere Reaktoren 10 oder Anlagen eingesetzt werden können und dass die Synthese mit regenerativer Energie und/oder mit regenerativem Ausgangsstoff AS betrieben werden kann. Bevorzugt ist der Einsatz regenerativer Energie und regenerativer Ausgangsstoffe AS.

Besonders vorteilhaft ist der Einsatz der Erfindung im Zusammenhang mit einem Verfahren zur Methanol-Wasser-Synthese, das bei niedrigen Drücken zwischen 10 und 150 bar (vorzugsweise bei ca. 80 bar) arbeitet.

Das Prinzip der Erfindung lässt sich auch auf Grossanlagen übertragen, eignet sich aber besonders für autarke Lokalanlagen zur Abwasserbehandlung.

Gemäß Erfindung dient $CO_2$ 101 als Ausgangsstoff und Kohlenstofflieferant für die Methanol-Wasser-Synthese im Reaktor 10. Als $CO_2$-Quellen dienen vorzugsweise: Steam-Reforming-Anlagen, Fermentationsanlagen, Feuerungsanlagen.

Je nach Synthesereaktion können z.B. kupfer-basierte Katalysatoren (z.B. CuO-Katalysatoren), oder Zinkoxid-Katalysatoren (z.B. ZnO-Katalysatoren), oder Chromoxid-Zinkoxid-Katalysatoren eingesetzt werden. Auch alle anderen bekannten Katalysatoren eignen sich für den Einsatz in einem Reaktor 10. Besonders geeignet sind Festbett-Katalysatoren oder Wirbelschicht-Katalysatoren. Der Katalysator kann auch einen geeigneten Träger (z.B. Karbon, Silikat, Aluminium (z.B. $Al_2O_3$) oder Keramik umfassen). Statt der erwähnten "metallischen" Katalysatoren kann auch ein organischer Katalysator eingesetzt werden.

Der Katalysator hat vorzugsweise bei allen Ausführungsformen eine Korn-, Kugel- oder Teilchengrösse zwischen 1 und 10 mm. Besonders bevorzugt ist eine Korn-, Kugel- oder Teilchengrösse zwischen 3 und 8 mm.

Im Folgenden werden weitere grundlegende Details des erfindungsgemässen Verfahrens und der entsprechenden Silicon-Fire Anlagen 100, 700, 800, 900 unter Verweis auf die Figuren 4 und 5 beschrieben. In Fig. 4 ist ein Schema einer Komplettanlage 800 gezeigt. Hier wird das Methanol 108 in einem Denitrifizierungstank 601 eingesetzt. Im Denitrifizierungstank 601 findet der Prozess 600 statt.

Fig. 5 zeigt das Prinzip der erfindungsgemässen Faulgas - Stromerzeugung und Silicon-Fire Methanol Produktion 900. Die gezeigte Silicon-Fire Mobilstation 603 erzeugt des Methanol 108, das im Schritt 600 zur Denitrifikation eingesetzt wird. Vorzugsweise liefert ein lokaler Stromgenerator 602 (vorzugsweise ein Stromgenerator 602 einer Faulgas - Stromerzeugungsanlage) elektrische Energie (Stromversorgung) an die Silicon-Fire Mobilstation 603. Ein $CO_2$-Abscheider 604 liefert das $CO_2$ für die Methanol-Synthese in der Silicon-Fire Mobilstation 603.

Um den Stickstoff aus dem Abwasser zu entfernen, wird vorzugsweise bei allen Ausführungsformen als letzter Behandlungsschritt eine Denitrifizierung unter Einsatz von Methanol 108 als Reduktionsmittel ausgeführt.

Das Reduktionsmittel (hier Methanol 108) sollte eine abbaubare organische Substanz sein, die von Bakterien verdaut werden kann und die das Wachstum neuer Bakterien ermöglicht. Das Methanol 108 erfüllt diese Vorgaben ideal, da, anders als in fossil hergestelltem Methanol, keine toxischen Verunreinigungen enthalten sein können.

Theoretisch ist 1,9 kg Methanol 108 erforderlich, um 1 kg Stickstoff aus dem Abwasser zu entfernen. In der Realität wird ca. 2,5 kg Methanol 108 pro kg Stickstoff gebraucht.

Vorzugsweise kommt bei allen Ausführungsformen eine eigene Stromerzeugung zum Einsatz, die z.B. durch ein Faulgas betriebenes Verbrennungsanlagen-Generator-System gewährleistet ist (siehe Fig. 5).

Durch die Erfindung ergeben sich neben ökologischen Vorteilen auch nachhaltige Kostenvorteile.

**Patentansprüche**

**1.** Verfahren zum Bereitstellen und Einsetzen einer methanolhaltigen Flüssigkeit (108) mit den folgenden Schritten:

- Bereitstellen (52; 104) eines Kohlenstoffdioxidgases (101) als Kohlenstofflieferant,
- Bereitstellen eines Wasserstoffgases (103),
- Bereitstellen eines gasförmigen Ausgangsstoffes (AS), der das Kohlenstoffdioxidgas (101) und Wasserstoffgas (103) umfasst,
- Einbringen des Ausgangsstoffes (AS) in einen Reaktor (10),
- Durchlaufen des Ausgangsstoffes (AS) durch eine Reaktionsstrecke des Reaktors (10), die mindestens teilweise mit einem Katalysator bestückt ist, um auf katalysator-synthetischem Weg die methanolhaltige Flüssigkeit (108) zu synthetisieren,
- Bereitstellen der methanolhaltigen Flüssigkeit (108) an einem ausgangsseitigen Ende (23) des Reaktors (10), wobei es sich bei der methanolhaltigen Flüssigkeit (108) um ein Gemisch (108) aus Methanol und Wasser handelt,
- Einsetzen der methanolhaltigen Flüssigkeit (108) in einem Denitrifizierungsprozess (600), um einen Stickstoffanteil aus Wasser, vorzugsweise aus Abwasser, zu entfernen.

**2.** Verfahren nach Anspruch 1, **dadurch gekennzeichnet, dass** ein Stromgenerator (602) mit Methangas und Kohlenstoffdioxidgas gespeist wird, um Strom und Rauchgas bereit zu stellen.

**3.** Verfahren nach Anspruch 2, **dadurch gekennzeichnet, dass** der Strom in dem Reaktor (10, 603) eingesetzt wird, um die methanolhaltige Flüssigkeit (108) zu erzeugen.

**4.** Verfahren nach Anspruch 2, **dadurch gekennzeichnet, dass** das Kohlenstoffdioxidgas abgeschieden (604) wird, um das Kohlenstoffdioxidgas dem Reaktor (10, 603) zuzuführen.

**5.** Verfahren nach einem der vorhergehenden Ansprüche, **dadurch gekennzeichnet, dass** Abwärme des Reaktors (10, 603) eingesetzt wird, um in einer Kläranlagen einen anderen Prozess zu unterstützen.

**6.** Verfahren nach einem der vorhergehenden Ansprüche, **dadurch gekennzeichnet, dass** es autark, d.h. ohne Zufuhr externer Mittel (Strom, Einsatzstoffe) ausgeführt wird.

**7.** Vorrichtung (100; 700; 800; 900), die zum Ausführen des Verfahrens nach einem der Ansprüche 1 bis 6 ausgelegt ist.

**8.** Kläranlage mit einer Faulgas - Stromerzeugungsanlage und mit einer Vorrichtung (100; 700; 800; 900) nach Anspruch 7 zur Methanol Produktion.

**9.** Kläranlage nach Anspruch 8, **dadurch gekennzeichnet, dass**

- ein lokaler Stromgenerator (602) als Teil der Faulgas - Stromerzeugungsanlage zum Einsatz kommt, um elektrische Energie an die Vorrichtung (100; 700; 800; 900) zur Methanol Produktion zu liefern,
- ein $CO_2$-Abscheider (604) zum Einsatz kommt, der das Kohlenstoffdioxidgas (101) für die Methanol-Synthese im Reaktor (10) liefert.

**10.** Kläranlage nach Anspruch 9, **dadurch gekennzeichnet, dass** der $CO_2$-Abscheider (604) mit dem Stromgenerator (602) so verbindbar ist, dass $CO_2$-haltige Rauchgas vom Stromgenerator (602) an den $CO_2$-Abscheider (604) übergeben werden kann.

Fig. 1

Fig. 2

10

21          21

17

H          D

14

16

23          23

Fig. 3

800

Absetzbecken

Abwasser

Belüftungstanks

Denitrifizierungstanks

Grobsiebe

erster
Sedimentationstank

In    Out

Sieben &
Abtrennen für die
Entsorgung

Rohschlamm

Komprimierte
Luft

Hydrogen-Methanol

601

108

Wasser

Schlamm zur
Weiterverarbeitung

Rückführung von
aktiviertem Schlamm

Fig. 4

Rauchgas
(N₂ + CO₂+ Dampf)

900

Klärschlamm

N₂ + O₂

N₂ + Dampf

Faulturm

Methan (CH₄)
+ CO₂

Stromgenerator

602

CO₂-
Abscheider

604

Faulschlamm

Stromversorgung

CO₂

Denitrifizierung

600

108

Wasser    603

Fig. 5

Europäisches Patentamt
European Patent Office
Office européen des brevets

## EUROPÄISCHER RECHERCHENBERICHT

**Nummer der Anmeldung**

EP 11 17 7709

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 2006 007139 A (TAKUMA KK) 12. Januar 2006 (2006-01-12) * Zusammenfassung; Abbildung * ----- | 1-10 | INV. B01J8/06 C02F11/04 C07C29/152 |
| X | GB 2 375 353 A (WATERGEM LTD [GB]) 13. November 2002 (2002-11-13) * Zusammenfassung * * Seite 1, Zeile 5 - Seite 2, Zeile 10 * * Seite 2, Zeile 29 - Zeile 30 * * Seite 3, Zeile 15 - Seite 4, Zeile 9 * * Seite 5, Zeile 11 - Seite 6, Zeile 29; Ansprüche; Abbildung * ----- | 1-10 | C07C31/04 C02F3/30 C12P5/02 |
| X | US 5 744 037 A (FUJIMURA HIROYUKI [JP] ET AL) 28. April 1998 (1998-04-28) * Zusammenfassung * * Spalte 5, Zeile 3 - Spalte 9, Zeile 13 * * Spalte 17, Zeile 44 - Spalte 18, Zeile 32; Ansprüche; Abbildungen 1, 2 * ----- | 1-10 | |
| A | US 2008/223783 A1 (SUTTON PAUL M [US]) 18. September 2008 (2008-09-18) * Zusammenfassung * * Absatz [0020] - Absatz [0024] * * Absatz [0030] - Absatz [0043] * * Absatz [0064]; Ansprüche; Abbildungen * ----- | 1-10 | RECHERCHIERTE SACHGEBIETE (IPC) B01J C02F C07C C12P |
| A | WO 98/36038 A1 (NORSKE STATS OLJESELSKAP [NO]; HALMOE TERJE M [NO]; MARTINSEN ALF S [N) 20. August 1998 (1998-08-20) * Zusammenfassung * * Seite 5, Zeile 25 - Seite 7, Zeile 4 * * Seite 8, Zeile 23 - Seite 13, Zeile 16 * * Ansprüche; Abbildung * ----- | 1-10 | |

Der vorliegende Recherchenbericht wurde für alle Patentansprüche erstellt

| Recherchenort | Abschlußdatum der Recherche | Prüfer |
|---|---|---|
| München | 14. Februar 2012 | Nazario, Luis |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 17 7709

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-02-2012

| Im Recherchenbericht angeführtes Patentdokument | Datum der Veröffentlichung | Mitglied(er) der Patentfamilie | Datum der Veröffentlichung |
|---|---|---|---|
| JP 2006007139 A | 12-01-2006 | JP 4563087 B2<br>JP 2006007139 A | 13-10-2010<br>12-01-2006 |
| GB 2375353 A | 13-11-2002 | KEINE | |
| US 5744037 A | 28-04-1998 | CN 1154949 A<br>US 5744037 A | 23-07-1997<br>28-04-1998 |
| US 2008223783 A1 | 18-09-2008 | GB 2460985 A<br>US 2008223783 A1<br>WO 2008115444 A2 | 23-12-2009<br>18-09-2008<br>25-09-2008 |
| WO 9836038 A1 | 20-08-1998 | AU 5886598 A<br>NO 970322 A<br>WO 9836038 A1 | 08-09-1998<br>27-07-1998<br>20-08-1998 |

EPO FORM P0461

Für nähere Einzelheiten zu diesem Anhang : siehe Amtsblatt des Europäischen Patentamts, Nr.12/82

**EP 2 438 980 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2010064948 W
- EP 11152947 A
- EP 11155310 A
- EP 11167622 A

- EP 0790226 B1
- WO 2010037441 A1
- EP 4483919 A2
- WO 2010069622 A1